# EUROPÄISCHE PATENTSCHRIFT

(11) **EP 2 655 366 B1**
(45) Veröffentlichungstag und Bekanntmachung des Hinweises auf die Patenterteilung: **19.08.2015**
(21) Anmeldenummer: 11851671.5
(22) Anmeldetag: 16.12.2011
(51) Int. Cl.: C07D 409/02, C07D 409/14, C07D 221/14, H01L 31/04, H01L 51/42, C07D 409/04, C07D 471/06, H01G 9/20, H01L 51/00

(54) **NAPTHALINMONOIMIDDERIVATE UND DEREN VERWENDUNG ALS PHOTOSENSIBILISATOREN IN SOLARZELLEN UND PHOTODETEKTOREN**
NAPHTALENE MONOIMIDE DERIVATIVES AND USE THEREOF AS PHOTOSENSITIZERS IN SOLAR CELLS AND PHOTODETECTORS
DÉRIVÉS DE MONOIMIDE DE NAPHTALÈNE ET LEUR UTILISATION COMME PHOTOSENSIBILISATEURS DANS DES CELLULES SOLAIRES ET PHOTODÉTECTEURS

(30) Priorität: 22.12.2010 EP 10196607
(43) Veröffentlichungstag der Anmeldung: 30.10.2013
(73) Patentinhaber: BASF SE, 67056 Ludwigshafen (DE)
(72) Erfinder: PSCHIRER, Neil Gregory, 55116 Mainz (DE); SCHÖNEBOOM, Jan, 68199 Mannheim (DE); EICKEMEYER, Felix, 69118 Heidelberg (DE); REICHELT, Helmut, 67435 Neustadt (DE); SENS, Rüdiger, 67069 Ludwigshafen (DE); BRUDER, Ingmar, 67376 Harthausen (DE)
(86) Internationale Anmeldenummer: PCT/IB2011/055750
(87) Internationale Veröffentlichungsnummer: WO 2012/085803

(56) Entgegenhaltungen:
- WO-A1-2008/132103
- WO-A1-2008/132103
- US-A1- 2005 214 568
- HUANG, XIAOMEI ET AL.: 'Novel dyes based on naphthalimide moiety as electron acceptor for efficient dye-sensitized solar cells.' DYES AND PIGMENTS Bd. 90, Nr. 3, 31 Januar 2011, Seiten 297 - 303, XP028169793
- HUANG, XIAOMEI ET AL.: 'Novel dyes based on naphthalimide moiety as electron acceptor for efficient dye-sensitized solar cells.' DYES AND PIGMENTS Bd. 90, Nr. 3, 31 Januar 2011, Seiten 297 - 303, XP028169793

## Beschreibung

Die vorliegenden Erfindung betrifft Verbindungen der Formeln Ia und Ib 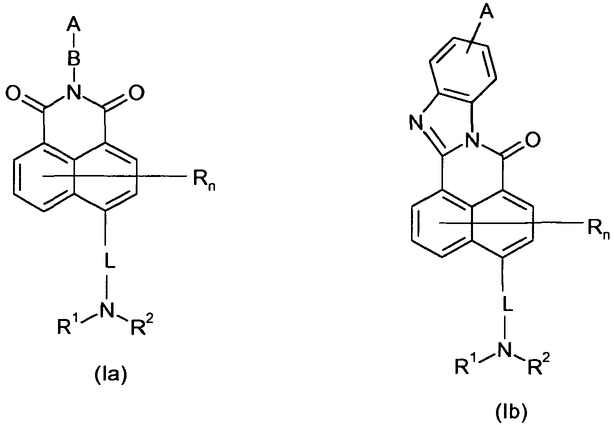 worin bedeuten
- R: gleiche oder verschiedene Reste Aryloxy, Arylthio, Hetaryloxy oder Hetarylthio,
- n: 0, 1, 2, 3, 4 oder 5,
- B: C₁-C₆-Alkylen oder 1,4-Phenylen, wobei der Phenylenrest ein- oder mehrfach durch Alkyl, Nitro, Cyano und/oder Halogen substituiert sein kann,
- A: -COOM, -SO₃M oder -PO₃M,
- M: Wasserstoff, Alkalimetallkation oder[NR'₄]⁺,
- R': Wasserstoff oder Alkyl, wobei die Reste R' gleich oder verschieden sein können,
- L: eine Brücke der Formeln

-Ar-, -Ar-Ar

oder

-Ar-Ar-Ar-

welche ein- oder mehrfach durch Phenyl, Alkyl, Alkoxy, Alkylthio und/oder -NR⁴R⁵ substituiert sein kann und in welcher Ar Aryl oder Hetaryl bedeutet, das mit gesättigten oder ungesättigten 5- bis 18-gliedrigen Ringen, die Heteroatome enthalten können, anelliert sein kann, wobei im Falle von zwei oder drei Ar diese gleich oder voneinander verschieden sind,
- R⁴, R⁵: unabhängig voneinander Wasserstoff, Alkyl, dessen Kohlenstoffkette durch eine oder mehrere Gruppierungen -O-, -S-, -CO-, -SO- und/oder -SO₂- unterbrochen sein kann, Aryl oder Hetaryl, das jeweils ein- oder mehrfach durch Alkyl, Alkoxy und/oder Alkylthio substituiert sein kann,
- R¹, R²: unabhängig voneinander Reste der Formeln IIa oder IIb 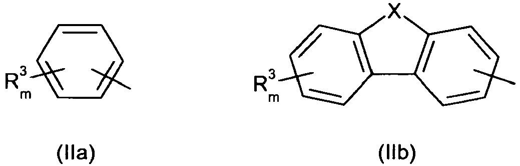
- R³: Phenyl, Alkyl, Alkoxy, Alkylthio oder -NR⁷R⁸,
- m: 0, 1, 2, 3 oder 4,
- X: C(R⁶R⁷)₂, NR⁸, Sauerstoff oder Schwefel und
- R⁶, R⁷, R⁸: unabhängig voneinander Wasserstoff, Alkyl, dessen Kohlenstoffkette durch eine oder mehrere Gruppierungen -O-, -S-, -CO-, -SO- und/oder -SO₂- unterbrochen sein kann, Aryl oder Hetaryl, das jeweils ein- oder mehrfach durch Alkyl, Alkoxy und/oder Alkylthio substituiert sein kann.

Weiter betrifft die vorliegende Erfindung die Verwendung von Verbindungen der Formeln Ia oder Ib oder Mischungen von Verbindungen der Formeln Ia und Ib und/oder Isomere oder Mischungen der Isomere der Verbindungen der Formeln Ia und Ib als Photosensibilisatoren in Solarzellen und Photodetektoren, sowie Solarzellen und Photodetektoren, welche solche Verbindungen der Formeln Ia oder Ib oder Mischungen von Verbindungen der Formeln Ia und Ib und/oder Isomere oder Mischungen der Isomere der Verbindungen der Formeln Ia und Ib als Photosensibilisatoren enthalten.

Die Direktumwandlung von Solarenergie in elektrische Energie in Solarzellen beruht auf dem inneren Photoeffekt eines Halbleitermaterials, d.h. der Erzeugung von Elek-tron-Loch-Paaren durch Absorption von Photonen und der Trennung der negativen und positiven Ladungsträger an einem p-n-Übergang oder einem Schottky-Kontakt. Die so erzeugte Photospannung kann in einem äußeren Stromkreis einen Photostrom bewirken, durch den die Solarzelle ihre Leistung abgibt.

Vom Halbleiter können dabei nur solche Photonen absorbiert werden, die eine Energie aufweisen, die größer als seine Bandlücke ist. Die Größe der Halbleiterbandlücke bestimmt also den Anteil des Sonnenlichts, der in elektrische Energie umgewandelt werden kann.

Dünne Schichten oder Filme von Metalloxiden stellen bekanntermaßen kostengünstige feste Halbleitermaterialien (n-Halbleiter) dar, jedoch liegt ihre Absorption aufgrund großer Bandlücken üblicherweise nicht im sichtbaren Bereich des elektromagnetischen Spektrums. Für die Anwendung in Solarzellen müssen die Metalloxide daher mit einem Photosensibilisator kombiniert werden, der im Wellenlängenbereich des Sonnenlichts, also bei 300 bis 2000 nm, absorbiert und im elektronischangeregten Zustand Elektronen in das Leitungsband des Halbleiters injiziert. Mithilfe eines zusätzlich in der Zelle eingesetzten Redoxsystems, das an der Gegenelektrode reduziert wird, werden Elektronen zum Sensibilisator zurückgeführt und dieser so regeneriert.

Von besonderem Interesse für die Anwendung in Solarzellen sind die Halbleiter Zinkoxid, Zinndioxid und insbesondere Titandioxid, die in Form nanokristalliner poröser Schichten zum Einsatz kommen. Diese Schichten weisen eine große Oberfläche auf, die mit dem Sensibilisator beschichtet wird, so daß eine hohe Absorption des Sonnenlichts erreicht wird.

Farbstoffsensibilisierte Solarzellen, die auf Titandioxid als Halbleitermaterial basieren, sind z.B. in US-A-4 927 721, Nature 353, S. 737-740 (1991) und US-A-5 350 644 sowie Nature 395, S. 583-585 (1998) und EP-A-1 176 646 beschrieben. Diese Solarzellen enthalten monomolekulare Filme aus Übergangsmetallkomplexen, insbesondere Rutheniumkomplexen, die über Säuregruppen an die Titandioxidschicht gebunden sind, als Sensibilisatoren und in gelöster Form vorliegende lod/lodid-Redoxsysteme bzw. amorphe organische p-Leiter auf Basis von Spirobifluorenen.

Als Sensibilisatoren wurden nicht zuletzt aus Kostengründen auch wiederholt metallfreie organische Farbstoffe vorgeschlagen.

So beschreibt beispielsweise US-A-6 359 211 für diesen Zweck Cyanin-, Oxazin-, Thiazin- und Acridinfarbstoffe, die über einen Alkylenrest gebundene Carboxylgruppen zur Fixierung an den Titandioxidhalbleiter aufweisen.

Perylen-3,4:9,10-tetracarbon-säurederivate als Sensibilisatoren werden in den japanischen Schriften JP-A-10-189065, 2000-243463, 2001-093589, 2000-100484 und 10-334954 und in New J. Chem. 26, S. 1155-1160 (2002) untersucht. Die Flüssigelektrolytsolarzellen auf Basis dieser Perylenderivate zeigten jedoch wesentlich geringere Wirkungsgrade als eine zum Vergleich mit einem Rutheniumkomplex sensibilisierte Solarzelle.

Zu den derzeit meist untersuchten Sensibilisatoren gehören Farbstoffe, welche über eine Cyanoacrylat-Ankergruppe verfügen. Beispielsweise wird von Kim, S.; Lee, J.K.; Kang, s.O.; Yum, j.H.; Fantacci, S.; DeAngelis, F.; Di Censo, D.; Nazeerruddin, M.K.; Grätzel, M. JACS 2006, 128, 16701 die Verbindung 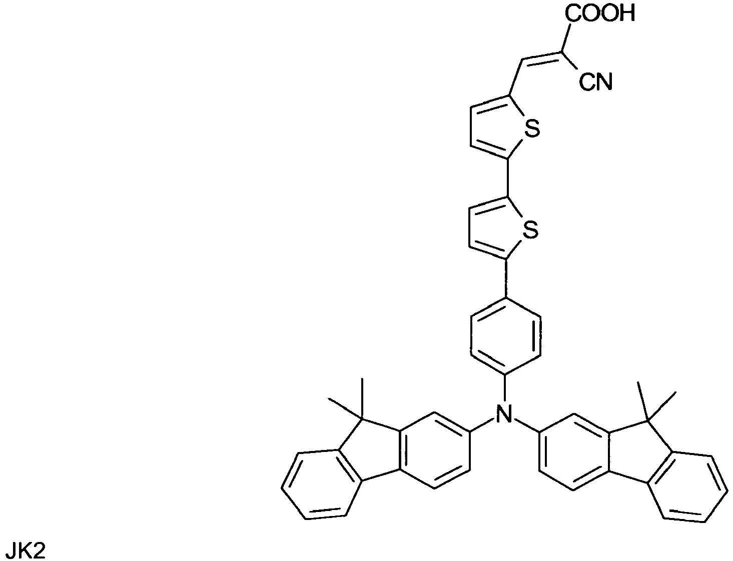 oder in Solar Energy Materials & Solar Cells 2009, 93, 1143 die Verbindung 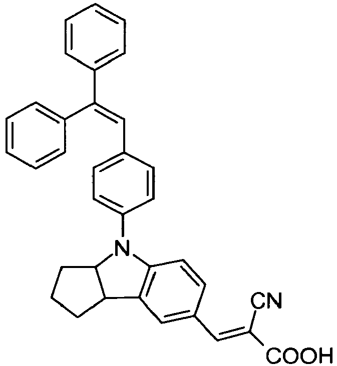 untersucht.

Gemäß Solar Energy Materials & Solar Cells 2009, 93, 1143 sind jedoch die Cyanoacrylat-Ankergruppen nicht ausreichend stabil, es wurde Decarboxylierung dieser Farbstoffe festgestellt und vermutet, dass die Ankergruppen gegenüber Licht instabil sind.

Farbstoffe mit Napthalenmonoimid-Ankergruppen sind in der Schrift WO 2008/132103 A1 beschrieben, doch absorbieren diese Verbindungen im kurzwelligen Spektralbereich des Sonnenlichts (absolutes Maximum bei etwa 450 nm) und sind daher für eine effiziente Absorption und Umwandlung des Sonnenlichts nicht geeignet.

Überrachenderweise wurden nun gefunden, dass Verbindungen der Fomeln Ia und Ib der vorliegenden Erfindung, welche Napthalinmonoimidgruppen als Ankergruppen enthalten, gute bis sehr gute Quanteneffizienzen bei sehr guten Stabilitäten in Farbstoffsolarzellen zeigen.

Demgemäß wurden die eingangs aufgeführten Verbindungen der Formeln Ia und Ib und deren Vewendung als Photosensibilisatoren in Solarzellen und Photodetektoren gefunden.

Im Sinne der vorliegenden Erfindung bedeutet Aryl, Arylrest, -einheit oder -gruppe insbesondere ein Rest mit einem Grundgerüst von 6 bis 30 Kohlenstoffatomen, bevorzugt 6 bis 18 Kohlenstoffatomen, der aus einem aromatischen Ring oder mehreren kondensierten aromatischen Ringen aufgebaut ist. Geeignete Grundgerüste sind zum Beispiel Phenyl, Benzyl, Naphthyl, Anthracenyl oder Phenanthrenyl. Dieses Grundgerüst kann unsubstituiert sein, d. h., dass alle Kohlenstoffatome, die substituierbar sind, Wasserstoffatome tragen, oder an einer, mehreren oder allen substituierbaren Positionen des Grundgerüsts substituiert sein. Geeignete Substituenten sind zum Beispiel Alkylreste, bevorzugt Alkylreste mit 1 bis 8 Kohlenstoffatomen, besonders bevorzugt Methyl, Ethyl, i-Propyl oder t-Butyl, Arylreste, bevorzugt C₆-Arylreste, die wiederum substituiert oder unsubstituiert sein können, Heteroarylreste, bevorzugt Heteroarylreste, die mindestens ein Stickstoffatom enthalten, besonders bevorzugt Pyridylreste, Alkenylreste, bevorzugt Alkenylreste, die eine Doppelbindung tragen, besonders bevorzugt Alkenylreste mit einer Doppelbindung und 1 bis 8 Kohlenstoffatomen, oder Gruppen mit Donor-oder Akzeptorwirkung. Unter Gruppen mit Donorwirkung sind Gruppen zu verstehen, die einen +l- und/oder +M-Effekt aufweisen, und unter Gruppen mit Akzeptorwirkung sind Gruppen zu verstehen, die einen -I- und/oder -M-Effekt aufweisen. Geeignete Gruppen, mit Donor- oder Akzeptorwirkung sind Halogenreste, bevorzugt F, CI, Br, besonders bevorzugt F, Alkylreste, Alkoxyreste, Aryloxyreste, Carbonylreste, Esterreste, Aminreste, Amidreste, CH₂F-Gruppen, CHF₂-Gruppen, CF₃-Gruppen, CN-Gruppen, Thiogruppen oder SCN-Gruppen. Ganz besonders bevorzugt tragen die Arylreste Substituenten ausgewählt aus der Gruppe bestehend aus Methyl, Ethyl, iso-Propyl, n-Propyl, n-Butyl, iso-Butyl, tert-Butyl, Aryloxy, Amin, Thiogruppen und Alkoxy, oder die Arylreste sind unsubstituiert. Bevorzugt ist der Arylrest oder die Arylgruppe ein Phenylrest, der gegebenenfalls mit mindestens einem der vorstehend genannten Substituenten substituiert ist. Besonders bevorzugt weist der Phenylrest keinen, einen, zwei oder drei der vorstehend genannten Substituenten auf.

Im Sinne der vorliegenden Erfindung bedeutet Heteroaryl, Heteroarylrest, -einheit oder -gruppe ein Rest mit 5 bis 30, bevorzugt mit 5 bis 18 Kohlenstoff- und Heteroatomen, der sich von den vorstehend genannten Arylresten dadurch unterscheidet, dass in dem Grundgerüst der Arylreste mindestens ein Kohlenstoffatom durch ein Heteroatom ersetzt ist. Bevorzugte Heteroatome sind N, O und S. Ganz besonders bevorzugt sind ein oder zwei Kohlenstoffatome des Grundgerüsts der Arylreste durch Heteroatome ersetzt. Insbesondere bevorzugt ist das Grundgerüst ausgewählt aus Systemen wie Pyridyl, Pyrimidyl, Pyrazyl und Triazolyl, und fünfgliedrigen Heteroaromaten wie Pyrrol, Furan, Thiophen, Imidazol, Pyrazol, Triazol, Oxazol und Thiazol. Das Grundgerüst kann an einer, mehreren oder allen substituierbaren Positionen des Grundgerüsts substituiert sein. Geeignete Substituenten sind dieselben, die bereits bezüglich der Arylgruppen genannt wurden.

Im Sinne der vorliegenden Erfindung leiten sich die Aryloxy-, Arylthio-, Hetaryloxy- und Hetarylthioreste formal von den zuvor genannte Aryl- und Heteroarylresten durch Anbindung eines Sauerstoff- bzw. Schwefelatoms an ein Kohlenstoffatom des Aryl- bzw. Heteroarylrestes ab.

Im Sinne der vorliegenden Erfindung bedeutet Alkyl, Alkylrest, -einheit oder-gruppe insbesondere ein Rest mit 1 bis 20 Kohlenstoffatomen, bevorzugt 1 bis 12 Kohlenstoffatomen zu verstehen. Dieser Alkylrest kann verzweigt oder unverzweigt sein und gegebenenfalls durch eine oder mehrere Gruppierungen -O-, -S-, -CO-, -SO- und/oder -SO₂- unterbrochen sein. Besonders bevorzugt ist Alkyl ausgewählt aus der Gruppe bestehend aus Methyl, Ethyl, i-Propyl, n-Propyl, i-Butyl, n-Butyl, t-Butyl, sec-Butyl, i-Pentyl, n-Pentyl, sec-Pentyl, neo-Pentyl, n-Hexyl, i-Hexyl und sec-Hexyl.

Im Sinne der vorliegenden Erfindung leiten sich Alkoxy- und Alkylthioreste formal von den zuvor genannte Alkylresten durch Anbindung eines Sauerstoff- bzw. Schwefelatoms an ein Kohlenstoffatom des Alkylrestes ab.

Im Sinne der vorliegenden Erfindung bedeutet Halogen bevorzugt F, CI oder Br, besonders bevorzugt F.

Im Sinne der vorliegenden Erfindung bedeutet Alkalimetallkation bevorzugt Li, Na, Cs und K, besonders bevorzugt Na.

Sofern die Brücke L ein- oder mehrfach durch Phenyl, Alkyl, Alkoxy, Alkylthio und/oder -NR4R5 substituiert ist, bedeutet dies, dass diese Substituenten an geeigneten Stellen der aromatischen und heteroaromatischen Gruppen Ar angebunden sind.

Enthält die Brücke L zwei oder drei Ar, so können diese gleich oder voneinander verschieden sein.

Als Beispiele für geeignete Ar seien genannt 1,4-, 1,3- und 1,2-Phenylen, 1,4- und 1,8-Naphthylen, 1,4- und 2,3-Pyrrylen, 2,5-, 2,4- und 2,3-Thienylen, 2,5-, 2,4- und 2,3-Furanylen, 2,3-, 2,4-, 2,5-, 2,6-, 3,4- und 3,5-Pyridinylen, 2,3-, 2,5-, 2,6-, 3,7-, 4,8-, 5,8- und 6,7-Chinolinylen, 2,7-, 3,6-, 4,5-, 2,6-, 3,7-, 4,7- und 4,8-Isochinolinylen, 1,4-[2,5-Di(tert.-butyl)]phenylen, 1,4-(2,5-Dihexyl)phenylen, 1,4-[2,5-Di(tert.-octyl)]phenylen, 1,4-(2,5-Didodecyl)phenylen und 1,4-[2,5-Di(2-dodecyl)]phenylen. Insbesondere kommen als Ar in Frage 1,4-Phenylen und 2,5-Thienylen.

Geeignete Brücken L sind beispielsweise: 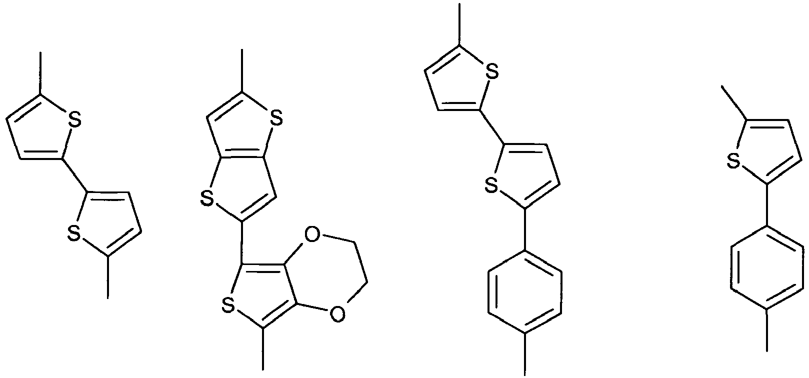 wobei bevorzugt sind:

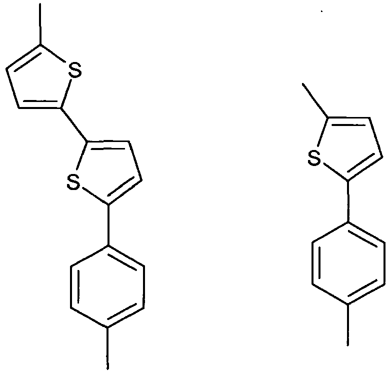

Bevorzugt sind erfindungsgemäße Verbindungen, in welchen in den Formeln Ia und Ib bedeuten:
- R: gleiche oder verschiedene Reste Aryloxy oder Arylthio, insbesondere Phenoxy oder Phenylthio
- n: 0, 1 oder 2,
- B: C₁-C₆-Alkylen, insbesondere -CH₂-, -(CH₂)₂-,und -(CH₂)₃-,
- A: -COOM,
- M: Wasserstoff oder Alkalimetallkation,
- L: eine Brücke der Formeln

-Ar-Ar-

oder

-Ar-Ar-Ar-

welche ein- oder mehrfach durch Phenyl, C₁-C₁₂-Alkyl, C₁-C₁₂-Alkoxy, C₁-C₁₂-Alkylthio und/oder -NR⁴R⁵ substituiert sein kann und in welcher Ar gleiches oder voneinander verschiedenes Aryl oder Hetaryl bedeutet, das mit gesättigten oder ungesättigten 5- bis 18-gliedrigen Ringen, die Heteroatome enthalten können, anelliert sein kann,
- R⁴, R⁵: unabhängig voneinander Wasserstoff, C₁-C₁₂-Alkyl, dessen Kohlenstoffkette durch eine oder mehrere Gruppierungen -O-, -S-, -CO-, -SO- und/oder -SO₂- unterbrochen sein kann,
- R¹, R²: unabhängig voneinander Reste der Formeln II'a und II'b 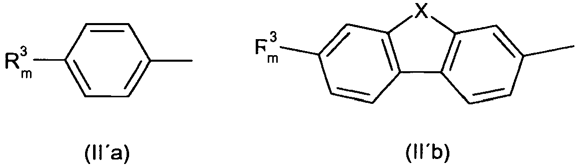
- R³: C₁-C₁₂-Alkoxy,
- m: 0 oder 1,
- X: C(R⁶R⁷)₂, NR⁸, Sauerstoff oder Schwefel und
- R⁶, R⁷, R⁸: unabhängig voneinander Wasserstoff, C₁-C₁₂-Alkyl, dessen Kohlenstoffkette durch eine oder mehrere Gruppierungen -O-, -S-, -CO-, -SO- und/oder -SO₂- unterbrochen sein kann.

Besonders bevorzugt sind erfindungsgemäße Verbindungen, in welchen in den Formeln Ia und Ib bedeuten
- n: 0,
- B: C₁-C₆-Alkylen, insbesondere -CH₂- und -(CH₂)₂-,
- A: -COOM,
- M: Wasserstoff oder Alkalimetallkation,
- L: eine Brücke der Formeln

-Ar-Ar-

oder

-Ar-Ar-Ar-

in welcher Ar gleiches oder voneinander verschiedenes Aryl oder Hetaryl bedeutet,
- R¹, R²: unabhängig voneinander Reste der Formeln II'a und II'b 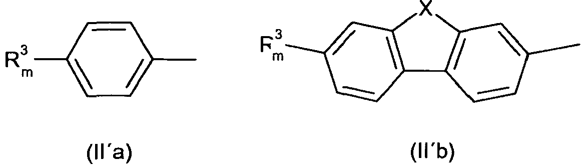
- R³: C₁-C₁₂-Alkoxy,
- m: 0 oder 1,
- X: C(R⁶R⁷)₂ und
- R⁶, R⁷: unabhängig voneinander Wasserstoff oder C₁-C₁₂-Alkyl.

Erfindungsgemäß sollen nicht nur die Verbindungen der Formeln Ia und Ib und deren bevorzugte Ausführungsformen umfasst sein, sondern auch deren Mischungen, deren Isomere sowie die Mischungen der Isomere.

Beispielsweise sind auch isomere Verbindungen der nachfolgend gezeigten Formel I*b 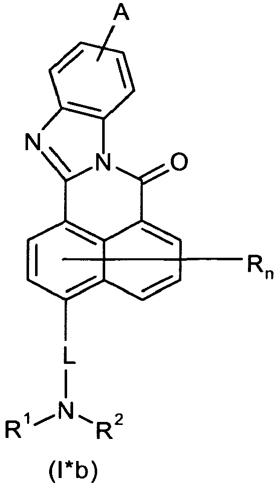 erfindungsgemäß eingeschlossen.

### Beispiele

### Beispiel 1:

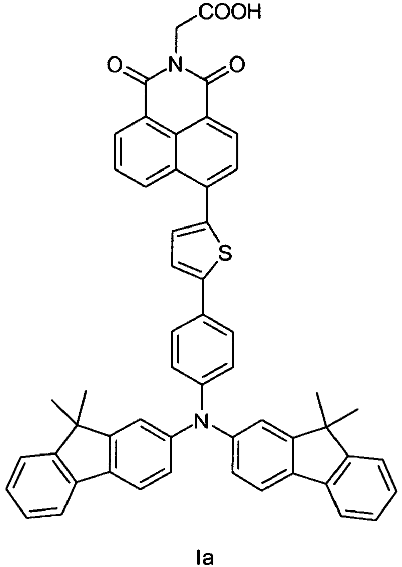

Die Herstellung erfolgte (entsprechend Ko et. al. Chemical Communications, 2004, 68-69) ausgehend von (4-Brom-phenyl)-bis-(9,9-dimethyl-9H-fluoren-2-yl)amin, welches zunächst mit Thiophen -2,5-diboronsäurester 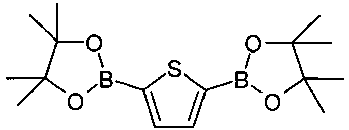 umgesetzt wurde (Schritt a). Anschließend folgte die Kupplung mit dem Methylester von N-(2-Carboxyethyl)-4-chlornaphtalimid und Entschützung der Estergruppe (Schritt b).

### Schritt a):

Eine Mischung aus 1,66 g (2,98 mmol) (4-Brom-phenyl)-bis-(9,9-dimethyl-9H-fluoren-2-yl)amin, 2,00 g (5,96 mmol) Thiophen -2,5-diboronsäurester, 0,82 g (5,96 mmol) K₂CO₃ gelöst in 5,2 mL H₂O/Ethanol 10:1, 92 mg (0,06 mmol) Pd(PPh₃)₄ und 10 mL Toluol wurde unter Stickstoff auf 85 °C erhitzt und 4 h bei dieser Temperatur gerührt.

Nach dem Abkühlen wurde das Reaktionsgemisch abgesaugt. Die Mutterlauge wurde gegen Dichlormethan/H₂O ausgeschüttelt. Der organischen Phase wurde das Lösungsmittel entzogen und der Rückstand getrocknet. Das Rohprodukt wurde ungereinigt im nächsten Reaktionsschritt eingesetzt.

### Schritt b):

Eine Mischung aus 2,00 g Rohprodukt Stufe a, 1,33 g (4,38 mmol) dem Methylester von N-(2-Carboxyethyl)-4-chlornaphtalinsäureimid, 50,4 mg (0,055 mmol) Pd2(dba)3, 1,43 g (4,38 mmol) Cs₂CO₃, 26,3 mg (0,13 mmol) Tributylphosphin und Dioxan wurde auf 90 °C erhitzt und 6 h bei dieser Temperatur gerührt. Nach dem Abkühlen wurde das Reaktionsgemisch abgesaugt und anschließend das Lösungsmittel der Mutterlauge entzogen. Das Rohprodukt wurde durch Säulenchromatographie mit n-Hexan/Essigester (2:1) aufgereinigt. Es wurden 70 mg der geschützten Verbindung erhalten.

Diese wurde mit 200 mg KOH in 10 mL VE-Wasser:THF 1:1 bei 60 °C einen Tag gerührt. Nach dem Abkühlen wurden 10 mL conc. HCl zugeben und 1 h nachgerührt. Dann wurde das Reaktionsgemisch auf VE-Wasser gegeben. Der Feststoff wurde abgesaugt, mit heißem VE-Wasser gewaschen und getrocknet.

Die Aufreinigung erfolgte durch Säulenchromatographie mit dem Eluent Dichlormethan:Ethanol 10:1 + 2 % Trimethylamin. Der erhaltene Feststoff wurde mit 50 %-iger Essigsäure bei 60 °C ausgerührt, abgesaugt und mit heißem VE-Wasser gewaschen. Nach dem Trocknen wurden 1,2 g eines roten Feststoffes erhalten.
Analytische Daten
¹H-NMR (500MHz, CDCI3, 25 °C): δ = 8,79 (d, 1H); 8,68 (d,1 H); 8,63 (d, 1 H); 7,88 (d, 1 H); 7,80 (q, 1 H), 7,66 (d, 2H); 7,63 (d, 2H); 7,58 (d, 2H); 7,40 (t, 3H); 7,33 (m, 3H); 7,27 (m, 4H); 7,23 (d, 2H); 7,15 (d, 2H); 5,02 (s, 2H); 1,43 (s, 12H)

### Beispiel 2:

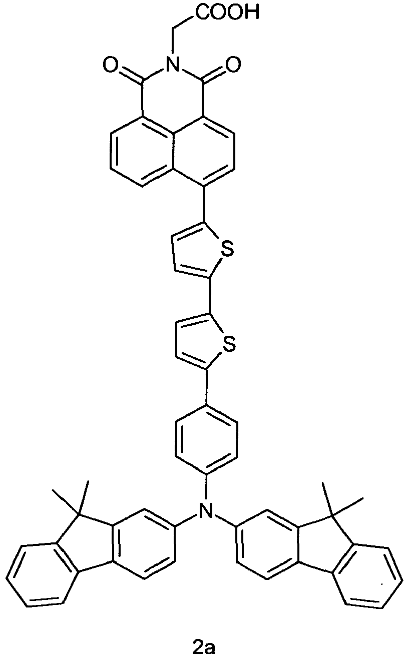

Die Herstellung erfolgte (entsprechend Ko et al., Chemical Communications, 2004, 68-69) ausgehend von (4-Brom-phenyl)-bis-(9,9-dimethyl-9H-fluoren-2-yl)amin, welches zunächst mit 2,2-Bisthiophen-5-boronsäureester umgesetzt wurde (Schritt a). Anschließend folgte die Bromierung (Schritt b) und danach die Kupplung mit dem 4-Boronsäureesternaphtalimid der Struktur 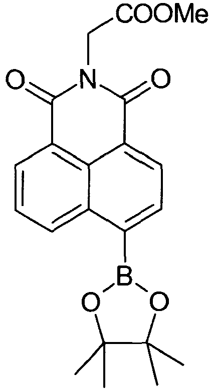 und Entschützung der Estergruppe (Schritt c).

### Schritt a:

Eine Mischung aus 1,67 g (3,00 mmol) (4-Brom-phenyl)-bis-(9,9-dimethyl-9H-fluoren-2-yl)amin, 1,80 mL (9,00 mmol) 5molarer NaOH und 10 mL Dioxan wurde 30 min mit Argon entgast. Dann wurden 54 mg (0,160 mmol) Pd[P(tBu)₃]₂ und 1,00 g (3,42 mmol) 2,2-Bisthiophen-5-boronsäureester 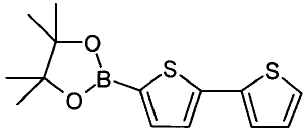 zugegeben, auf 85 °C erhitzt und über das Wochenende gerührt. Nach dem Abkühlen wurde das Reaktionsgemisch auf Eiswasser gegeben. Anschließend wurde mit Dichlormethan extrahiert und der organischen Phase das Lösungsmitel entzogen. Der Rückstand wurde in wenig Tetrahydrofuran gelöst. Dazu wurde Methanol zugeben bis ein Feststoff ausfiel. Dieser wurde abgesaugt, mit wenig Methanol gewaschen und getrocknet. Es wurden 1,40 g eines gelben Feststoffes erhalten, was einer Ausbeute von 73 % entspricht.
Analytische Daten:
¹H-NMR (500MHz, DMSO, 25 °C): δ = 7,77 (m, 4H); 7,62 (d, 2H); 7,51 (d, 3H); 7,41 (d, 1H); 7,31 (m, 8H); 7,11 (m, 3H); 7,05 (d, 2H); 1,37 (s, 12H)

### Schritt b:

Zu einer Mischung aus 1,40 g (2,18 mmol) Stufe a und 20 mL Dimethylformamid (DMF) wurde bei 0 - 5 °C eine Lösung aus 466 mg (2,62 mmol) n-Bromsuccimid und 10 mL DMF zugetropft. Bei dieser Temperatur wurde dann 15 min nachgerührt und anschließend 10 mL verdünnte Natriumthiosulfatlösung zugegeben. Das Reaktionsgemisch wurde auf 150 mL VE-Wasser gegeben und mit Methyl-tert-butylether (MTBE) extrahiert. Der organischen Phase wurde das Lösungsmittel entzogen und der Rückstand getrocknet. Es wurden 1,20 g eines gelben Feststoffes erhalten, was einer Ausbeute von 76 % entspricht.
Analytische Daten:
¹H-NMR (500MHz, DMSO, 25 °C): δ = 7,77 (m, 4H); 7,62 (d, 2H); 7,51 (d, 2H); 7,42 (d, 1 H); 7,31 (m; 7H); 7,23 (d, 1 H); 7,17 (d, 1 H); 7,11 (d, 2H); 7,06 (d, 1 H); 1,37 (s, 12H)

### Schritt c:

Eine Mischung aus 1,00 g (1,4 mmol) Stufe b, 0,84 mL (4,2 mmol) 5molare NaOH und 15 mL Dioxan wurde 30 min mit Argon entgast. Dann wurden 24 mg (0,05 mmol) Pd[P(tBu)₃]₂ und 0,97 g (1,6 mmol) des oben gezeigten 4-Boronsäureesternaphtalimids (65 %) zugegeben, auf 85 °C erhitzt und 1 Tag gerührt. Nach dem Abkühlen wurde das Reaktionsgemisch auf Eiswasser gegeben und mit Dichlormethan extrahiert. Der organischen Phase wurde das Lösungsmittel entzogen. Der Rückstand wurde mittels Säulenchromatographie mit dem Eluens Dichlormethan:Methanol 4:1 aufgereinigt.

Es wurde geschütztes Zielprodukt erhalten, welches mit THF:Wasser 1:1 und 1 g KOH über Nacht bei 65 °C gerührt wurde. Nach dem Abkühlen wurde das Reaktionsgemisch auf Wasser gegeben und mit 15 mL conc. HCl versetzt. Es wurde 1 h bei Raumtemperatur gerührt. Dann wurde der Feststoff abgesaugt, gewaschen und getrocknet. Das Rohprodukt wurde mittels Säulenchromatographie mit dem Eluens Dichlormethan:Methanol 4:1 aufgereinigt. Man erhielt 330 mg eines roten Feststoffes, was einer Ausbeute von 26 % entspricht.
Analytische Daten:
¹H-NMR (500MHz, DMSO, 25 °C): δ = 8,80 (d, 1 H); 8,61 (d, 1 H); 8,55 (d, 1 H); 7,99 (q, 2H); 7,76 (g, 4H); 7,65 (d, 2H); 7,55 (q, 2H); 7,51 (d, 2H); 7,47 (d, 2H); 7,30 (m, 6H); 7,12 (d, 2H); 7,06 (d, 2H); 4,75 (s, 2H); 1,34 (s, 12H)

### Beispiel 3:

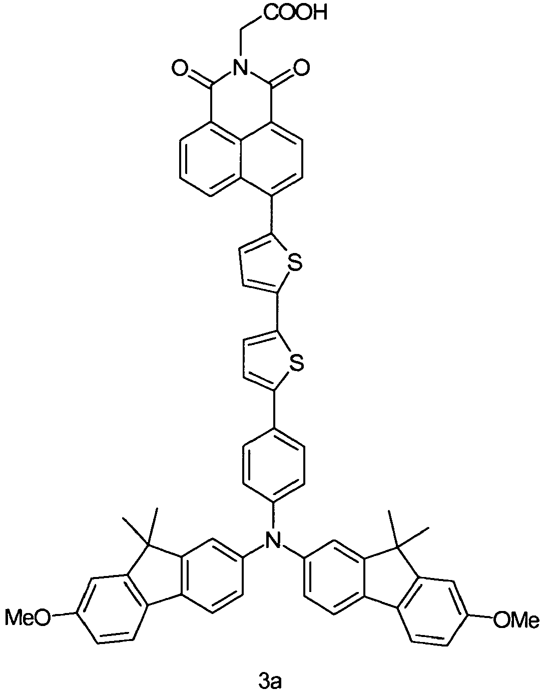

Die Herstellung erfolgte ausgehend vom Diarylamin, welches zunächst mit Bisthiophen-5-boronsäureester umgesetzt wurde (Schritt a). Anschließend erfolgte Bromierung (Schritt b) und danach Kupplung mit 2-Boronsäureestemaphtalinsäureimid (Schritt c):

### Schritt a:

Eine Mischung aus 1,80 g (2,9 mmol) Diarylamin, 15 mL Dioxan und 1,74 mL (8,7 mmol) 5molarer NaOH wurde 30 min mit Argon entgast. Dann wurden 51 mg (0,1 mmol) Pd[P(tBu)₃]₂ und 0,91 g (3,11 mmol) Bisthiophen-5-boronsäureester zugegeben, der Ansatz auf 85 °C erhitzt und über das Wochenende gerührt. Nach dem Abkühlen wurde das Reaktionsgemisch auf Eiswasser gegeben und mit Dichlormethan extrahiert. Der organischen Phase wurde das Lösungsmittel entzogen, der Rückstand in wenig THF gelöst und mit Methanol versetzt. Der ausgefallene Feststoff wurde abgesaugt und getrocknet. Die Aufreinigung erfolgte mittels Säulenchromatographie mit dem Eluens n-Hexan + 2 % Essigester. Man erhielt 1,70 g eines gelben Feststoffes, was einer Ausbeute von 85 % entspricht.
Analytische Daten:
¹H-NMR (500MHz, DMSO, 25 °C): δ = 7,41 (d, 4H); 7,34 (d, 2H); 7,26 (d, 1 H); 7,14 (d, 1H); 7,08 (d, 1 H); 7,04 (d, 1 H); 6,99 (d, 2H); 6,86 (d, 3H); 6,81 (d, 2H); 6,76 (d, 2H); 6,65 (d, 2H); 3,56 (s, 6H); 1,11 (s, 12H)

### Schritt b:

Zu einer Mischung aus 1,6 g (2,3 mmol) Stufe a und 30 mL DMF wurde bei 0 - 5 °C eine Lösung aus 490 mg (2,40 mmol) n-Bromsuccimid und 10 mL DMF zugetropft. Bei dieser Temperatur wurde dann 15 min nachgerührt und anschließend 10 mL verdünnte Natriumthiosulfatlösung zugegeben. Das Reaktionsgemisch wurde auf 150 mL VE-Wasser gegeben. Der Feststoff wurde abgesaugt, gewaschen und getrocknet. Es wurden 1,62 g eines gelben Feststoffes erhalten, was einer Ausbeute von 90 % entspricht.
Analytische Daten:
¹H-NMR (500MHz, DMSO, 25 °C): δ = 7,66 (d, 4H); 7,58 (d, 2H); 7,40 (d, 1 H); 7,30 (d, 1H); 7,26 (d, 2H); 7,23 (d, 1H); 7,16 (d, 1H); 7,11 (d, 2H); 7,07 (d, 2H); 7,01 (d, 2H); 6,90 (d, 2H); 3,81 (s, 6H); 1,36 (s, 12H)

### Schritt c:

Eine Mischung aus 1,00 g (1,3 mmol) Stufe b, 0,78 mL (3,9 mmol) 5molare NaOH und 15 mL Dioxan wurde 30 min mit Argon entgast. Dann wurden 26 mg (0,05 mmol) Pd[P(tBu)₃]₂ und 0,59 g (1,5 mmol) der Verbindung 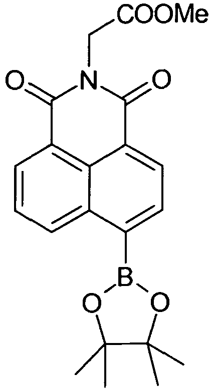 zugegeben, der Ansatz auf 85 °C erhitzt und 1 Tag gerührt. Nach dem Abkühlen wurde das Reaktionsgemisch auf Eiswasser gegeben und mit Dichlormethan extrahiert. Der organischen Phase wurde das Lösungsmittel entzogen. Der Rückstand wurde zweimals mittels Säulenchromatographie mit dem Eluens Dichlormethan:Methanol 4:1 aufgereinigt. Man erhielt 630 mg eines roten Feststoffes, was einer Ausbeute von 68 % entspricht.
Analytische Daten:
¹H-NMR (500MHz, DMSO, 25 °C): δ = 8,75 (d, 1 H); 8,58 (d, 1 H); 8,51 (d, 1 H); 7,96 (m, 2H); 7,65 (d, 4H); 7,62 (d, 2H); 7,53 (s, 2H); 7,45 (s, 2H); 7,26 (d, 2H); 7,11 (d, 2H); 7,07 (d, 2H); 7,02 (d, 2H); 6,90 (d, 2H); 4,51 (s, 2H); 3,81 (s, 6H); 1,36 (s, 12H)

### Beispiel 4:

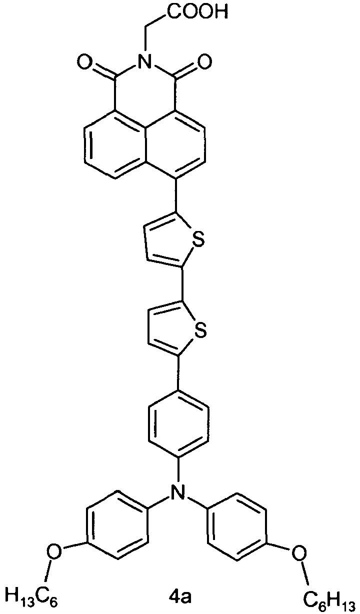

Die Herstellung erfolgt ausgehend von 4-Hexyloxybrombenzol, welches mit p-Hexyloxyanilin umgesetzt wurde (Schritt a). Das entstandene Amin wurde mit 1,4-Dibrombenzol weiter umgesetzt (Schritt b). Danach wurde mit 2,2-Bisthiophen-5-boronsäureester gekuppelt (Schritt c) und bromiert (Schritt d). Zum Schuss erfolgte die Kupplung mit 2-Boronsäureestemaphtalimid und die Entschützung der Estergruppe (Schritt e).

### Schritt a:

Eine Mischung aus 20 g (78 mmol) 4-Hexyloxyanilin, 18 g (93 mmol) p-Hexyloxybrombenzol, 0,87 g (3,9 mmol) Palladium-II-acetat, 3,1 g (5,8 mmol) DPEphos, 12 g (125 mmol) Natrium-tert-butylat und 100 mL Toluol wurde auf 100 °C erhitzt und einen Tag gerührt. Nach dem Abkühlen wurde dem Reaktionsgemisch das Lösungsmittel entzogen. Die Aufreinigung des Rohproduktes erfolgte mittels Säulenchromatographie mit dem Eluens Dichlormethan:Hexan 2:1. Es wurden 19,7 g eines weißen Feststoffes erhalten, was einer Ausbeute von 68 % entspricht.
Analytische Daten:
¹H-NMR (500 MHz, CD2Cl2, 25 °C): δ = 6,91 (d, 4H); 6,79 (d, 4H); 5,35 (s, 1 H); 3,90 (t, 4H); 1,74 (m, 4H); 1,45 (m, 4H); 1,34 (m, 8H); 0,91 (t,6H);

### Schritt b:

Eine Mischung aus 5,4 g (14,6 mmol) Stufe a, 6,9 g (29,2 mmol) 1,4-Dibrombenzol, 275 mg (0,30 mmol) Pd₂(dba)₃, 405 mg (0,73 mmol) DPPF, 2,80 g (29,2 mmol) Natrium-tert-butylat und 40 mL Toluol wurde auf 90 °C erhitzt und 2 Tage gerührt. Nach dem Abkühlen wurde dem Reaktionsgemisch das Lösungmittel entzogen. Die Aufreinigung erfolgte mittels Säulenchromatographie mit dem Eluens Hexan:Dichlormethan 4:1. Es wurden 5,6 g eines hellen Öls erhalten, was einer Ausbeute von 72 % entspricht.
Analytische Daten:
¹H-NMR (500MHz, DMSO, 25 °C): δ = 7,29 (d, 2H); 6,99 (d, 4H); 6,89 (d, 4H); 6,66 (d, 2H); 3,92 (t, 4H); 1,69 (m, 4H); 1,40 (m, 4H); 1,30 (m, 8H); 0,88 (t, 6H)

### Schritt c:

Eine Mischung aus 1,6 g Stufe b (3,1 mmol), 15 mL Dioxan und 1,86 mL (9,3 mmol) 5molarer NaOH wurde 30 min mit Argon entgast. Dann wurden 51 mg (0,11 mmol) Pd[P(tBu)₃]₂ und 0,98 g (3,31 mmol) 2,2-Bisthiophen-5-boronsäureester zugegeben, der Ansatz auf 85 °C erhitzt und über das Wochenende gerührt. Nach dem Abkühlen wurde das Reaktionsgemisch auf Eiswasser gegeben und mit Dichlormethan extrahiert. Der organischen Phase wurde das Lösungsmittel entzogen. Die Aufreinigung erfolgte mittels Säulenchromatographie mit dem Eluens n-Hexan + 2% Essigester. Man erhielt 1,62 g eines gelben Feststoffes, was einer Ausbeute von 86 % entspricht.
Analytische Daten:
¹H-NMR (500MHz, DMSO, 25 °C): δ = 7,48 (m, 3H); 7,30 (m, 2H); 7,25 (d, 1 H); 7,09 (q, 1 H); 7,03 (q, 4H); 6,92 (q, 4H); 6,76 (q, 2H); 3,94 (t, 4H); 1,70 (m, 4H); 1,41 (m, 4H); 1,31 (m, 8H); 0,88 (t, 6H)

### Schritt d:

Zu einer Mischung aus 1,2 g (2,00 mmol) Stufe c und 30 mL DMF wurde bei 0 - 5 °C eine Lösung aus 430 mg (2,40 mmol) n-Bromsuccimid und 10 mL DMF getropft. Bei dieser Temperatur wurde dann 15 min nachgerührt und anschließend 10 mL verdünnte Natriumthiosulfatlösung zugegeben. Das Reaktionsgemisch wurde auf 150 mL VE-Wasser gegeben, mit MTBE extrahiert und der organischen Phase das Lösungsmittel entzogen. Es wurden 1,0 g eines gelben Feststoffes erhalten, was einer Ausbeute von 73 % entspricht.
Analytische Daten:
¹H-NMR (500MHz, DMSO, 25 °C): δ = 7,46 (d, 2H; 7,29 (d, 1 H; 7,27 (d, 1 H; 7,21 (d, 1 H; 7,14 (d, 1 H; 7,03 (d, 4H; 6,92 (d, 4H; 6,75 (d, 2H; 3,94 (t, 4H; 1,70 (m, 4H; 1,41 (m, 4H; 1,31 (m, 8H; 0,98 (t, 6H)

### Schritt e:

Eine Mischung aus 0,90 g (1,3 mmol) Stufe d, 0,78 mL (3,9 mmol) 5molarer NaOH und 15 mL Dioxan wurde 30 min mit Argon entgast. Dann wurden 22 mg (0,04 mmol) Pd[P(tBu)₃]₂ und 0,91 g (1,5 mmol) der Verbindung 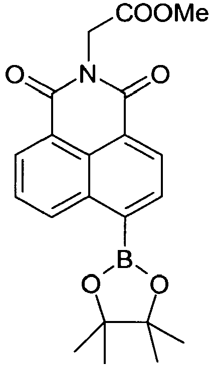 zugegeben, der Ansatz auf 85 °C erhitzt und 1 Tag gerührt. Nach dem Abkühlen wurde das Reaktionsgemisch auf Eiswasser gegeben, mit Dichlormethan extrahiert und der organischen Phase das Lösungsmittel entzogen. Der Rückstand wurde zweimals mittels Säulenchromatographie mit dem Eluens Dichlormethan:Methanol 4:1 + 1% Triethylamin aufgereinigt. Es wurde geschütztes Zielprodukt erhalten, welches mit THF:Wasser 1:1 und 1 g KOH über Nacht bei 65 °C gerührt wurde. Nach dem abkühlen wurde das Reaktionsgemisch auf Wasser gegeben und mit 15 mL conc. HCl versetzt. Es wurde 1 h bei Raumtemperatur gerührt. Dann wurde mit Dichloremthan extrahiert und der organischen Phase anschließend das Lösungsmittel entzogen. Das Rohprodukt wurde mittels Säulenchromatographie mit dem Eluens Dichlormethan: Methanol 4:1 + 2 % Triethylamin aufgereinigt. Man erhielt 610 mg eines roten Feststoffes, was einer Ausbeute von 54 % entspricht.
Analytische Daten:
¹H-NMR (500MHz, DMSO, 25 °C): δ = 8,78 (d, 1H); 8,60 (d, 1H); 8,53 (d, 1H); 7,97 (m, 2H); 7,53 (m, 4H); 7,41 (d, 1 H); 7,34 (d, 1 H); 7,03 (d, 4H); 6,92 (d, 4H); 6,77 (d, 2H); 4,72 (s, 2H); 3,93 (t, 4H); 1,70 (m, 4H); 1,41 (m, 4H); 1,31 (m, 8H); 0,88 (t, 6h)

### Beispiel 5 (Verbindung fiel als Gemisch der Isomeren 5a und 5b an):

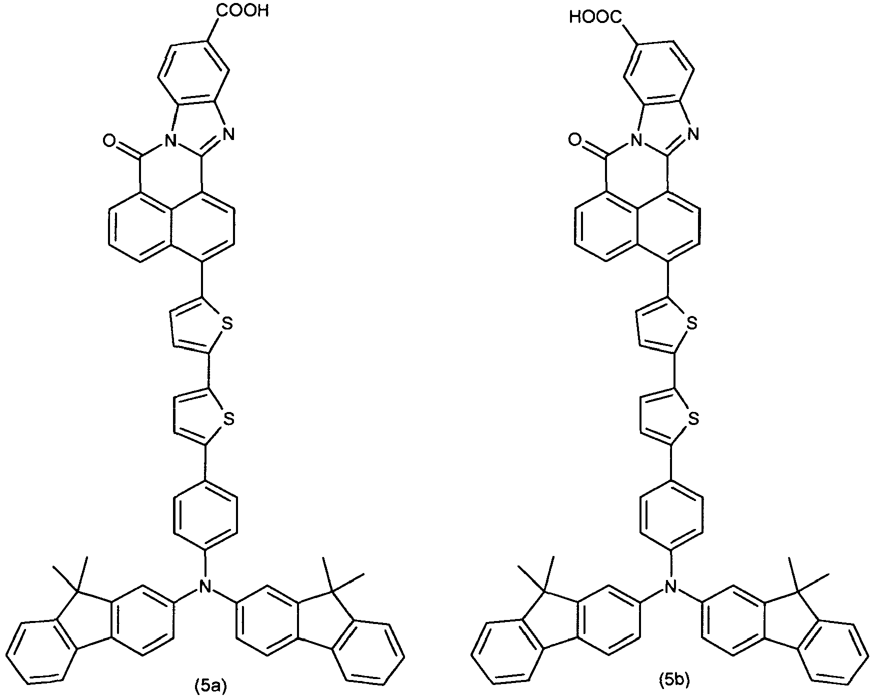

[4-(5'-Bromo-[2,2']bisthiophenyl-5-yl)-phenyl]-bis-(9,9-dimethyl-9H-fluoren-2-yl)-amin (450 mg, 0,62 mmol) wurde in Dioxan gelöst (15 ml), 5molare NaOH (0,4 mL, 1,9 mmol) zugegeben und der Ansatz 30 min mit Argon entgast. Dann wurden Pd[P(tBu)₃]₂ (10 mg, 0,02 mmol) und 6-(4,4,5,5-Tetramethyl-[1,3,2]dioxaborolan-2-yl)benzo[de]isochromen-1,3-dion (330 mg, 1 mmol) eintragen und der Ansatz wurde über Nacht bei 85 °C gerührt. Nach dem Abkühlen wurde auf Eiswasser gegeben, mit Dichlormethan extrahiert und die organische Phase eingeengt. Man erhielt 490 mg eines orangenen Feststoffes.
Maldi-MS: M+ = 837,27

In Chinolin (40 ml) wurden 6-(5'-{4-[Bis-(9,9-dimethyl-9H-fluoren-2-yl)-amino]-phenyl[2,2']bithiophenyl-5-yl)-benzo[de]isochromen-1,3-dion (400 mg, 0,48 mmol), Zinkacetat (88 mg, 0,22 mmol) und 3,4-Diaminobenzoesäure (220 mg, 1,44 mmol) eingetragen. Der Ansatz wurde auf 220 °C erhitzt und 7 h gerührt. Nach dem
Abkühlen wurde auf 6%ige HCl gefällt, der Feststoff abgesaugt und mit heißem Wasser und wenig Ethanol gewaschen. Säulenchromatographie mit Dichlormethan:Methanol 5:1 ergab 98 mg (21 %) eines orangenen Feststoffes.
Maldi-MS: M+=953,24

### Anwendungsbeispiele:

Als Substrate wurden mit fluordotiertem Zinnoxid (FTO) beschichtete Glasplatten der Abmessung 25 mm x 15 mm x 3 mm (Nippon Sheet Glass) eingesetzt, die nacheinander mit Glasreiniger (RBS 35), vollentsalztem Wasser und Aceton jeweils 5 min im Ultraschallbad behandelt, dann 10 Minuten in Iso-Propanol gekocht und im Stickstoffstrom getrocknet wurden.

Zur Herstellung der festen TiO₂-Barriereschicht wurde ein Spraypyrolyseverfahren verwendet, wie in Peng et al., Coord. Chem. Rev. 248 (2004), 1479, beschrieben. Auf diese feste TiO₂-Barriereschicht wurde im Siebdruckverfahren die TiO₂-Past DSL 18NR-T (Dyesol) aufgedruckt. Die Paste bestand aus TiO₂-Partikeln mit einem Durchmesser von ca. 25 nm, welche in einer Terpineol/Ethylcellulose-Mischung dispergiert wurden. Nach dem Druckprozess wurde die Paste für 5 Minuten bei 80 °C getrocknet. Anschließend folgte die 30-minütige Sinterung bei 450 °C. Die resultirende nanoporöse TiO₂ Schicht besaß eine Schichtdicke von 1,8 µm.

Zur elektrischen Isolation zwischen Metallrückelektroden und Arbeitselektroden wurden neben der TiO₂-Schicht längsseitig jeweils Streifen von Polyimid (Pyrolin Polyimide Coating, Supelco) aufgesetzt und für 15 min bei 200 °C im Trockenschrank ausgehärtet.

Nach Herausnehmen aus dem Trockenschrank wurde die Probe auf 80 °C abgekühlt, 16 h lang in eine 5 x 10-4 molare ethanolische Lösung des Hydroxamsäuresalzes (das Salz wurde durch Umsetzung der kommerziell erhältlichen Hydroxamsäure mit Natronlauge erhalten) 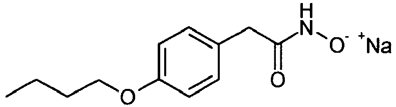 getaucht, anschließend herausgenommen, kurz mit EtOH gewaschen und dann 1 h lang in eine 5 x 10-4 molare Lösung des erfindungsgemäßen Farbstoffs bzw. eine Lösung der Vergleichsverbindung JK2 in Dichlormethan gelegt. Die aus der Lösung herausgenommene Probe wurde anschließend mit reinem Lösungsmittel (hier Dichlormethan) abgespült und im Stickstoffstrom getrocknet. Auf die getrocknete Probe wurde eine p-Leiter-Lösung aufgeschleudert. Die Lösung bestand aus: 0,16 M spiro-MeOTAD (Merck) in Chlorbenzol und 0,3 M LiN(SO₂CF₃)₂ (Aldrich) in Cyclohexanon im Verhältnis 15:1 und 2,5 Gewichtsprozent V2O5 bezogen auf spiro-MeOTAD. 125 µl dieser Lösung wurden auf die Probe aufgebracht und 60 s einwirken gelassen. Danach wurde die überstehende Lösung 30 s bei 2000 rmp abgeschleudert.

Die Metallrückelektrode wurde durch thermische Metallverdampfung im Vakuum aufgebracht. Dazu versah man die Probe mit einer Maske, um 8 voneinander getrennte Rückelektroden mit den Abmessungen 0,13 cm² auf die aktive Region aufzubringen. Dazu wurde Ag mit einer Rate von 3,0 - 3,5 nm/s bei einem Druck von ca. 5*10-5 mbar verdampft, so dass eine Schichtdicke von 200 nm resultiert.

Die Quanteneffizienz (IPCE = Incident Photon-to-current Conversion Efficiency) wurde mit einer 75-Watt-Xenon-Bogenlampe (LOT-Oriel), einem 1/8 m Monochromator (SpectraPro-2150i; Acton Research Corporation), einem Transimpedanzverstärker (Aescusoft GmbH Automation) und einem Lock-in-Verstärker 7265 (Signal Recovery) gemessen.

Es wurden Strom/Spannungs-Kennlinien bei einer Beleuchtungsstärke von 100 mW/cm² (Xenon-Lampe (LOT-Oriel) mit AM1.5 Filter) durch Variation der Spannung zwischen -0,6 V und +1,0 V und Messung des resultierenden Kurzschluss-Stromes erhalten. Die Daten für verschiedene erfindungsgemäße Verbindungen und die Verbindung JK2 gemäß des Standes der Technik sind in Tabelle 1 gezeigt (I_{SC}: Kurzschluss-Strom; V_{OC}: Spannung bei offenem Stromkreis; FF: Füllfaktor; ETA: Wirkungsgrad).

**Tabelle I**

| Farbstoff | I_{sc}[mA/cm²] | V_{oc}[mV] | FF[%] | ETA[%] |
|---|---|---|---|---|
| 2a | -7,8 | 820 | 63 | 3,9 |
| 4a | -7,5 | 840 | 61 | 3,8 |
| 3a | -7,4 | 840 | 59 | 3,6 |
| 3a (Toluol)* | -7,7 | 860 | 64 | 4,1 |
| 5a/b | -7.8 | 900 | 42 | 2,9 |
| JK2 | -9,2 | 860 | 58 | 4,6 |

| | | | | |
|---|---|---|---|---|
| *: "3a (Toluol)" bedeutet, dass der Farbstoff nicht wie üblich aus Dichlormethan-, sondern aus Toluol-Lösung aufgebracht wurde. | | | | |

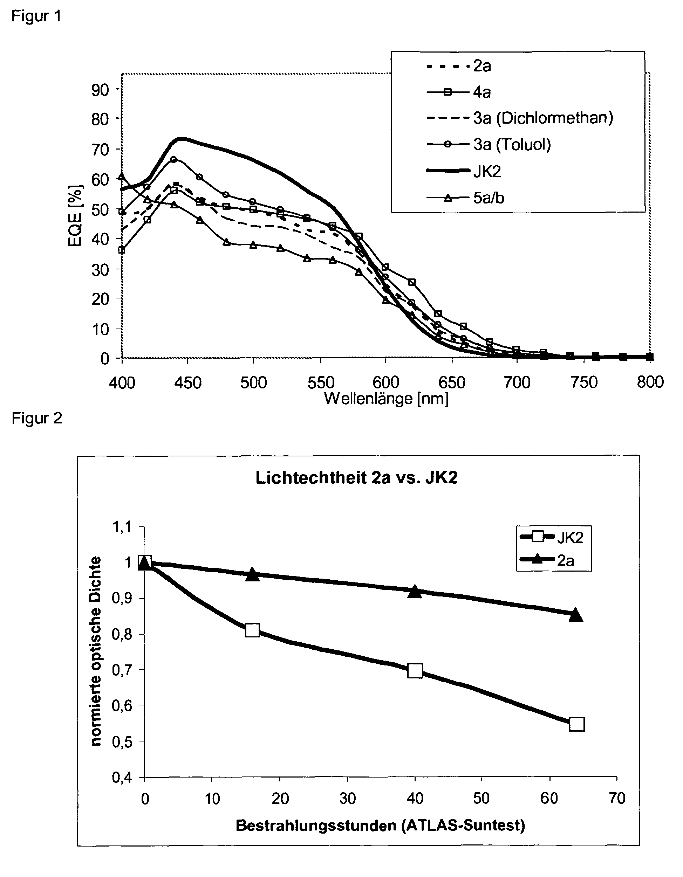

Figur 1 zeigt die EQE-Werte der erfindungsgemäßen Verbindungen und der Verbindung JK2 des Standes der Technik in Abhängigkeit von der Wellenlänge.

Figur 2 zeigt einen Vergleich der Lichtechtheit des erfindungsgemäßen Farbstoffes der Verbindung 2a im Vergleich zum Farbstoff JK2 des Standes der Technik jeweils auf TiO₂. Die Bestrahlungsreihe wurde nach 2h "Light Soaking" im Suntester begonnen.

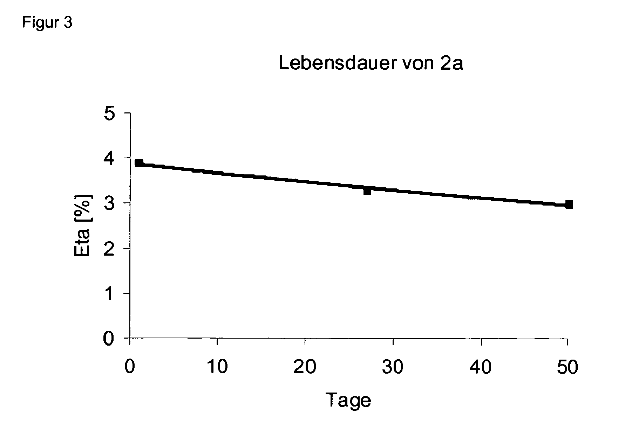

Figur 3 zeigt die Effizienz einer kurzgeschlossenen Solarzellen mit Farbstoff 2a in Abhängigkeit von der Zeit. Zur Bestimmung des Wirkungsgrads wurde die Strom/Spannungs-Kennlinie mit einem Source Meter Model 2400 (Keithley Instruments Inc.) unter Bestrahlung mit einer Leistung von 100 mW/cm2 (Xenon-Lampe (LOT-Oriel) mit AM 1.5 Filter) aufgenommen.

## Patentansprüche

1. Verbindungen der Formeln la und Ib 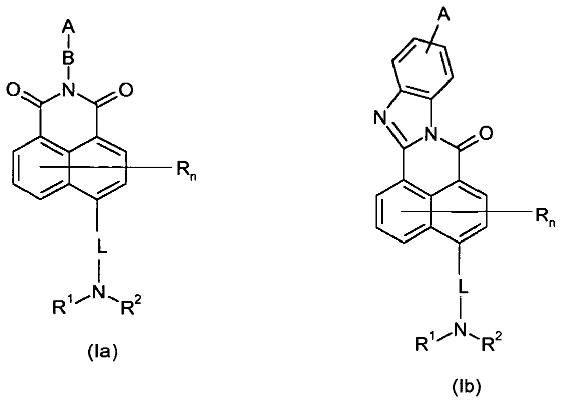 worin bedeuten
R gleiche oder verschiedene Reste Aryloxy, Arylthio, Hetaryloxy oder Hetarylthio,
n 0, 1, 2, 3, 4 oder 5,
B C₁-C₆-Alkylen oder 1,4-Phenylen, wobei der Phenylenrest ein- oder mehrfach durch Alkyl, Nitro, Cyano und/oder Halogen substituiert sein kann,
A -COOM, -SO₃M oder -PO₃M,
M Wasserstoff, Alkalimetallkation oder [NR'₄]⁺,
R' Wasserstoff oder Alkyl, wobei die Reste R' gleich oder verschieden sein können,
L eine Brücke der Formeln
-Ar-, -Ar-Ar
oder
-Ar-Ar-Ar-
welche ein- oder mehrfach durch Phenyl, Alkyl, Alkoxy, Alkylthio und/oder - NR⁴R⁵ substituiert sein kann und in welcher Ar Aryl oder Hetaryl bedeutet, das mit gesättigten oder ungesättigten 5- bis 18-gliedrigen Ringen, die Heteroatome enthalten können, anelliert sein kann, wobei im Falle von zwei oder drei Ar diese gleich oder voneinander verschieden sind,
R⁴, R⁵ unabhängig voneinander Wasserstoff, Alkyl, dessen Kohlenstoffkette durch eine oder mehrere Gruppierungen -O-, -S-, -CO-, -SO- und/oder -SO₂- unterbrochen sein kann, Aryl oder Hetaryl, das jeweils ein- oder mehrfach durch Alkyl, Alkoxy und/oder Alkylthio substituiert sein kann,
R¹, R² unabhängig voneinander Reste der Formeln IIa oder IIb 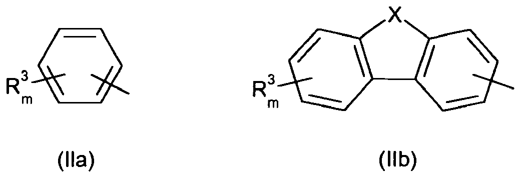
R³ Phenyl, Alkyl, Alkoxy, Alkylthio oder -NR⁷R⁸,
m 0, 1, 2, 3 oder 4,
X C(R⁶R⁷)₂, NR⁸, Sauerstoff oder Schwefel und
R⁶, R⁷, R⁸ unabhängig voneinander Wasserstoff, Alkyl, dessen Kohlenstoffkette durch eine oder mehrere Gruppierungen -O-, -S-, -CO-, -SO- und/oder -SO₂- unterbrochen sein kann, Aryl oder Hetaryl, das jeweils ein- oder mehrfach durch Alkyl, Alkoxy und/oder Alkylthio substituiert sein kann.

2. Verbindungen nach Anspruch 1, **dadurch gekennzeichnet, dass** in den Formeln la und Ib bedeuten
R gleiche oder verschiedene Reste Aryloxy oder Arylthio,
n 0, 1 oder 2,
B C₁-C₆-Alkylen,
A -COOM,
M Wasserstoff oder Alkalimetallkation,
L eine Brücke der Formeln
-Ar-Ar
oder
-Ar-Ar-Ar-
welche ein- oder mehrfach durch Phenyl, C₁-C₁₂-Alkyl, C₁-C₁₂-Alkoxy, C₁-C₁₂-Alkylthio und/oder -NR⁴R⁵ substituiert sein kann und in welcher Ar gleiches oder voneinander verschiedenes Aryl oder Hetaryl bedeutet, das mit gesättigten oder ungesättigten 5- bis 18-gliedrigen Ringen, die Heteroatome enthalten können, anelliert sein kann,
R⁴, R⁵ unabhängig voneinander Wasserstoff, C₁-C₁₂-Alkyl, dessen Kohlenstoffkette durch eine oder mehrere Gruppierungen -O-, -S-, -CO-, -SO- und/oder -SO₂-unterbrochen sein kann,
R¹, R² unabhängig voneinander Reste der Formeln II'a und II'b 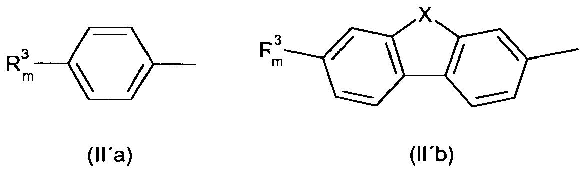
R³ C₁-C₁₂-Alkoxy,
m 0 oder 1,
X C(R⁶R⁷)₂, NR⁸, Sauerstoff oder Schwefel und
R⁶, R⁷, R⁸ unabhängig voneinander Wasserstoff, C₁-C₁₂-Alkyl, dessen Kohlenstoffkette durch eine oder mehrere Gruppierungen -O-, -S-, -CO-, -SO- und/oder -SO₂-unterbrochen sein kann.

3. Verbindungen nach Anspruch 1, **dadurch gekennzeichnet, dass** in den Formeln la und Ib bedeuten
n 0,
B C₁-C₆-Alkylen,
A -COOM,
M Wasserstoff oder Alkalimetallkation,
L eine Brücke der Formeln
-Ar-Ar
oder
-Ar-Ar-Ar- in welcher Ar gleiches oder voneinander verschiedenes Aryl oder Hetaryl bedeutet,
R¹, R² unabhängig voneinander Reste der Formeln II'a und II'b 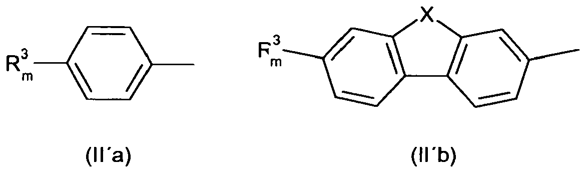
R³ C₁-C₁₂-Alkoxy,
m 0 oder 1,
X C(R⁶R⁷)₂ und
R⁶, R⁷ unabhängig voneinander Wasserstoff oder C₁-C₁₂-Alkyl.

4. Verwendung von Verbindungen der Formeln la oder Ib oder Mischungen von Verbindungen der Formeln la und Ib gemäß Anspruch 1, 2 oder 3 und/oder Isomere oder Mischungen der Isomere der Verbindungen der Formeln la und Ib gemäß Anspruch 1, 2 oder 3 als Photosensibilisatoren in Solarzellen und Photodetektoren.

5. Solarzellen und Photodetektoren, enthaltend Verbindungen der Formeln la oder Ib oder Mischungen von Verbindungen der Formeln la und Ib gemäß Anspruch 1, 2 oder 3 und/oder Isomere oder Mischungen der Isomere der Verbindungen der Formeln la und Ib gemäß Anspruch 1, 2 oder 3 als Photosensibilisatoren.

## Claims

1. A compound of the formula Ia or Ib 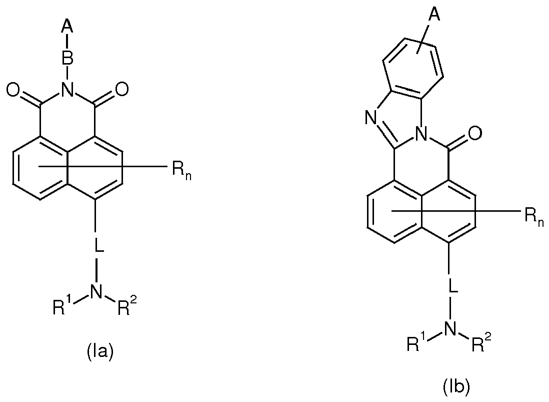 in which
R are identical or different aryloxy, arylthio, hetaryloxy or hetarylthio radicals,
n is 0, 1, 2, 3, 4 or 5,
B is C₁-C₆-alkylene or 1,4-phenylene, where the phenylene radical may be mono- or polysubstituted by alkyl, nitro, cyano and/or halogen,
A is -COOM, -SO₃M or -PO₃M,
M is hydrogen, an alkali metal cation or [NR'₄]⁺,
R' is hydrogen or alkyl, where the R' radicals may be the same or different,
L is a bridge of the formula
-Ar-, -Ar-Ar
or
-Ar-Ar-Ar-
which may be mono- or polysubstituted by phenyl, alkyl, alkoxy, alkylthio and/or - NR⁴R⁵, and in which Ar is aryl or hetaryl which may be fused to saturated or unsaturated 5- to 18-membered rings which may comprise heteroatoms which may be the same or different in the case of two or three Ar,
R⁴, R⁵ are each independently hydrogen, alkyl whose carbon chain may be interrupted by one or more -0-, -S-, -CO-, -SO- and/or - SO- moieties, aryl or hetaryl, each of which may be mono- or polysubstituted by alkyl, alkoxy and/or alkylthio,
R¹, R² are each independently radicals of the formula IIa or IIb 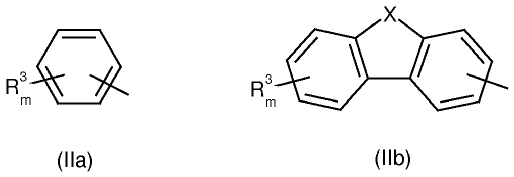
R³ is phenyl, alkyl, alkoxy, alkylthio or - NR⁷R⁸,
m is 0, 1, 2, 3 or 4,
X is C(R⁶R⁷)₂, NR⁸, oxygen or sulfur and
R⁶, R⁷, R⁸ are each independently hydrogen, alkyl whose carbon chain may be interrupted by one or more -0-, -S-, -CO-, -SO- and/or - SO₂- moieties, aryl or hetaryl, each of which may be mono- or polysubstituted by alkyl, alkoxy and/or alkylthio.

2. A compound according to claim 1, wherein, in the formulae Ia and Ib,
R are identical or different aryloxy or arylthio radicals,
n is 0, 1 or 2,
B is C₁-C₆-alkylene,
A is -COOM,
M is hydrogen or an alkali metal cation,
L is a bridge of the formula
-Ar-Ar or -Ar-Ar-Ar-
which may be mono- or polysubstituted by phenyl, C₁-C₁₂-alkyl, C₁-C₁₂-alkoxy, C₁-C₁₂-alkylthio and/or -NR⁴R⁵, and in which Ar is identical or different aryl or hetaryl which may be fused to saturated or unsaturated 5- to 18-membered rings which may comprise heteroatoms,
R⁴, R⁵ are each independently hydrogen, C₁-C₁₂-alkyl whose carbon chain may be interrupted by one or more -0-, -S-, -CO-, -SO- and/or -SO₂- moieties,
R¹, R² are each independently radicals of the formulae II'a and II'b 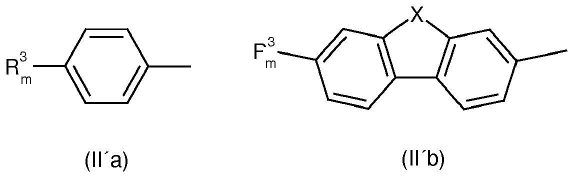
R³ is C₁-C₁₂-alkoxy,
m is 0 or 1,
X is C(R⁶R⁷)₂, NR⁸, oxygen or sulfur and
R⁶, R⁷, R⁸ are each independently hydrogen, C₁-C₁₂-alkyl whose carbon chain may be interrupted by one or more -0-, -S-, -CO-, -SO- and/or -SO₂- moieties.

3. A compound according to claim 1, wherein, in the formulae Ia and Ib,
n is 0,
B is C₁-C₆-alkylene,
A is -COOM,
M is hydrogen or an alkali metal cation,
L is a bridge of the formula
-Ar-Ar
or
-Ar-Ar-Ar-
in which Ar is identical or different aryl or hetaryl,
R¹, R⁶ are each independently radicals of the formulae II'a and II'b 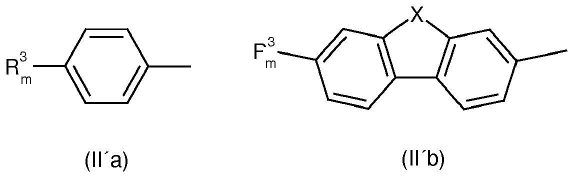
R³ is C₁-C₁₂-alkoxy,
m is 0 or 1,
X is C(R⁶R⁷)₂ and
R⁶, R⁷ are each independently hydrogen or C₁-C₁₂-alkyl.

4. The use of compounds of the formula Ia or Ib or mixtures of compounds of the formulae Ia and Ib according to claim 1, 2 or 3 and/or isomers or mixtures of the isomers of the compounds of the formulae Ia and Ib according to claim 1, 2 or 3 as photosensitizers in solar cells and photodetectors.

5. A solar cell or photodetector comprising compounds of the formula Ia or Ib or mixtures of compounds of the formulae Ia and Ib according to claim 1, 2 or 3 and/or isomers or mixtures of the isomers of the compounds of the formulae Ia and Ib according to claim 1, 2 or 3 as photosensitizers.

## Revendications

1. Composés de formules Ia et Ib 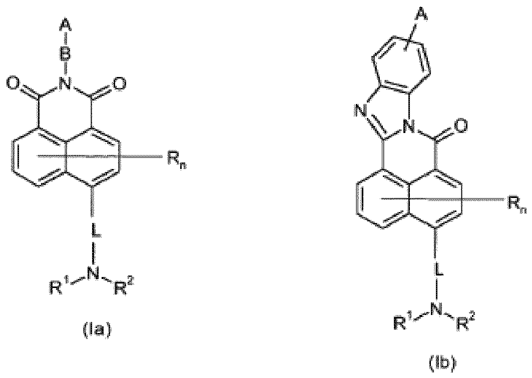 dans lesquelles
R représente des radicaux identiques ou différents aryloxy, arylthio, hétéroaryloxy ou hétéroarylthio,
n représente 0, 1, 2, 3, 4 ou 5,
B représente un groupe alkylène en C₁-C₆ ou 1,4-phénylène, le radical phénylène pouvant être une ou plusieurs fois substitué par alkyle, nitro, cyano et/ou halogéno,
A représente -COOM, -SO₃M ou -PO₃M,
M représente un atome d'hydrogène, un cation de métal alcalin ou [NR'₄]⁺,
R' représente un atome d'hydrogène ou un groupe alkyle, les radicaux R' pouvant être identiques ou différents,
L représente un pont de formules
-Ar-, -Ar-Ar- ou -Ar-Ar-Ar-
qui peut être une ou plusieurs fois substitué par phényle, alkyle, alcoxy, alkylthio et/ou NR⁴R⁵ et dans lequel Ar représente un groupe aryle ou hétéroaryle qui peut être soudé à des cycles saturés ou insaturés à 5 à 18 chaînons, qui peuvent contenir des hétéroatomes, dans le cas de deux ou trois Ar ceux-ci pouvant être identiques ou différents,
R⁴, R⁵ représentent indépendamment l'un de l'autre un atome d'hydrogène, un groupe alkyle dont la chaîne carbonée peut être interrompue par un ou plusieurs groupements -0-, -S-, -CO-, -SO- et/ou -SO₂-, un groupe aryle ou hétéroaryle qui peut dans chaque cas être une ou plusieurs fois substitué par alkyle, alcoxy et/ou alkylthio,
R¹, R⁹ représentent indépendamment l'un de l'autre des radicaux de formule IIa ou IIb 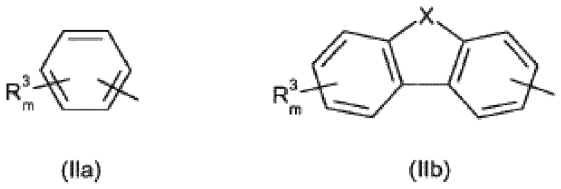
R³ représente un groupe phényle, alkyle, alcoxy, alkylthio ou NR⁷R⁸,
m représente 0, 1, 2, 3 ou 4,
X représente C(R⁶R⁷)₂, NR⁸, un atome d'oxygène ou de soufre et
R⁶, R⁷, R⁸ représentent chacun indépendamment un atome d'hydrogène, un groupe alkyle dont la chaîne carbonée peut être interrompue par un ou plusieurs groupements -0-, -S-, -CO-, -SO-et/ou -SO₂-, un groupe aryle ou hétéroaryle qui peut dans chaque cas être une ou plusieurs fois substitué par alkyle, alcoxy et/ou alkylthio.

2. Composés selon la revendication 1, **caractérisé en ce que** dans les formules Ia et Ib
R représente des radicaux identiques ou différents aryloxy ou arylthio,
n représente 0, 1 ou 2,
B représente un groupe alkylène en C₁-C₆,
A représente -COOM,
M représente un atome d'hydrogène ou un cation de métal alcalin,
L représente un pont de formules
-Ar-Ar- ou -Ar-Ar-Ar-
qui peut être une ou plusieurs fois substitué par phényle, alkyle en C₁-C₁₂, alcoxy en C₁-C₁₂, alkyl(C₁-C₁₂)thio et/ou NR⁴R⁵ et dans lequel Ar représente un groupe aryle ou hétéroaryle, identique ou chaque fois différent, qui peut être soudé à des cycles saturés ou insaturés à 5 à 18 chaînons, qui peuvent contenir des hétéroatomes,
R⁴, R⁵ représentent indépendamment l'un de l'autre un atome d'hydrogène, un groupe alkyle en C₁-C₁₂ dont la chaîne carbonée peut être interrompue par un ou plusieurs groupements -O-, -S-, -CO-, -SO- et/ou -SO₂-,
R¹, R² représentent indépendamment l'un de l'autre des radicaux de formules II'a et II'b 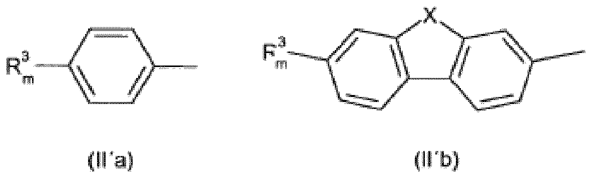
R³ représente un groupe alcoxy en C₁-C₁₂,
m représente 0 ou 1,
X représente C(R⁶R⁷)₂, NR⁸, un atome d'oxygène ou de soufre et
R⁶, R⁷, R⁸ représentent chacun indépendamment un atome d'hydrogène, un groupe alkyle en C₁-C₁₂ dont la chaîne carbonée peut être interrompue par un ou plusieurs groupements -0-, -S-, -CO-, -SO- et/ou -SO₂-.

3. Composés selon la revendication 1, **caractérisé en ce que** dans les formules Ia et Ib
n représente 0,
B représente un groupe alkylène en C₁-C₆,
A représente -COOM,
M représente un atome d'hydrogène ou un cation de métal alcalin,
L représente un pont de formules
-Ar-Ar- ou -Ar-Ar-Ar-
dans lesquelles Ar représente un groupe aryle ou hétéroaryle identique ou chaque fois différent,
R¹, R² représentent indépendamment l'un de l'autre des radicaux de formules II'a et II'b 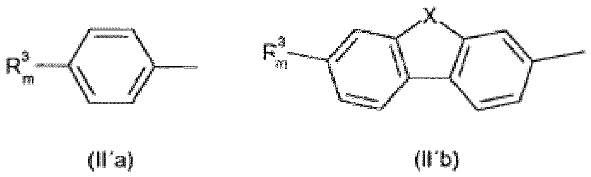
R³ représente un groupe alcoxy en C₁-C₁₂,
m représente 0 ou 1,
X représente C(R⁶R⁷)₂ et
R⁶, R⁷ représentent indépendamment l'un de l'autre un atome d'hydrogène ou un groupe alkyle en C₁-C₁₂.

4. Utilisation de composés de formule Ia ou Ib ou de mélanges de composés de formules Ia et Ib selon la revendication 1, 2 ou 3 et/ou d'isomères ou de mélanges des isomères des composés de formules Ia et Ib selon la revendication 1, 2 ou 3 en tant que photosensibilisateurs dans des cellules solaires et des photodétecteurs.

5. Cellules solaires et photodétecteurs, contenant des composés de formule Ia ou Ib ou des mélanges de composés de formules Ia et Ib selon la revendication 1, 2 ou 3 et/ou des isomères ou des mélanges des isomères des composés de formules Ia et Ib selon la revendication 1, 2 ou 3 en tant que photosensibilisateurs.
